# EUROPEAN PATENT APPLICATION

(11) **EP 2 165 680 A1**
(43) Date of publication of application: **24.03.2010**
(21) Application number: 08164511.1
(22) Date of filing: 17.09.2008
(51) Int. Cl.: A61F 2/30, A61L 26/00, A61L 27/50

(54) **Textile Implant**

(71) Applicant: University of Bern, 3012 Bern (CH); Bischoff Textil AG, 9001 St. Gallen (CH)
(72) Inventor: Bischoff, Bernard, 9000 St. Gallen (CH); Ferguson, Stephen, 3251 Ruppoldsried (CH)
(74) Representative: Liebetanz, Michael

(57) **Abstract**

An internal body implant comprises a three-dimensional textile structure having a top surface (T) and a bottom surface (B). The textile structure comprises multiple yarns being interwoven with each other. The top surface (T) is provided with active properties, such as adhesive properties and/or properties promoting adhesion and/or properties promoting ongrowth and/or ingrowth of proteins, cells and/or biological tissues. Said active properties are absent at the bottom surface (B).

## Description

### Technical field of the invention

The invention relates to a textile implant according to the preamble of claim 1.

### Prior Art

Implants for internal implantation in the human or animal body are known from prior art. EP 1 787 604 for example discloses an implant for sealing and healing a defect in an annulus of an intervertebral disc. Such an implant is attachable to the vertebral body and extends over the defect (e.g. a fissure) in the annulus such that the defect is covered by the implant.

Although tests of the implant according to EP 1 787 604 have shown that the handling for the medical practitioner is easy and facile, there is a need for a more sophisticated structure in terms of attaching the implant and/or preventing unwanted local tissue adhesion.

### Summary of the invention

An object of the present invention is to provide a textile implant, which provides more sophisticated attachment properties or adhesive properties. Additionally such an implant shall prevent unwanted local tissue adhesion.

This object is achieved by the implant of claim 1. An internal body implant or membrane comprises a three-dimensional textile structure having a top surface and a bottom surface. The textile structure comprises multiple yarns being interwoven with each other. The top surface is provided with active properties, such as adhesive properties and/or properties promoting adhesion and/or properties promoting ongrowth and/or ingrowth of proteins, cells and/or biological tissues. Said active properties are absent at the bottom surface.

Such an implant is advantageous as the top surface is able to be in an adhesive contact with the injured organ or tissue, whereas the bottom surface is a non-adhesive contact with the neighbouring organs or tissue.

Preferably the bottom surface is provided with non-active properties. This enhances the non-adhesive contact between the implant and the respective organ or tissue.

Preferably the active properties are applied to at least one section of the top surface wherein said section is defined as having a smaller area than the top surface. Such a structure enhances the implant further as it can be attached to more precisely and as it prevents unwanted tissue adhesion in the respective section(s).

Preferably the active properties are applied to the entire top surface. Such a structure is advantageous, if the whole implant shall be adhesive.

Preferably the implant comprises active yarns having said active properties and carrier yarns wherein the active yam faces the top surface and wherein the carrier yam faces the bottom surface. Such an embroidered structure with said active yarns and non-active yarns can be produced in a cost-effective manner.

Preferably the active yam and the carrier yam are provided in the form of a plurality of filling threads and a plurality of chaining threads which are interwoven with each other, wherein the active properties are applied to the filling threads and/or to the chaining threads which are arranged towards to top surface.

The active yam is preferably composed predominately or exclusively of yam materials that promote adhesion, ongrowth and/or ingrowth of cells and/or biological tissue.

Preferably the surface of the active yam is pre-coated with a coating material that promotes adhesion, ongrowth and/or ingrowth of cells and/or biological tissue. With such a structure all the yarns of the textile implant may be the same, such that they have similar or equal mechanical properties.

Preferably the top surface is coated predominately or exclusively with a coating material that promotes adhesion, ongrowth and/or ingrowth of cells and/or biological tissue.

Preferably the non-active yam is composed predominately or exclusively of yam materials that inhibits adhesion, ongrowth and/or ingrowth of cells and/or biological tissue.

The surface of the non-active yam is preferably pre-coated with a coating material that inhibits adhesion, ongrowth and/or ingrowth of cells and/or biological tissue.

Preferably the bottom surface is coated predominately or exclusively with a coating material that inhibits adhesion, ongrowth and/or ingrowth of cells and/or biological tissue.

### Brief description of the drawings

The drawings will be explained in greater detail by means of a description of an exemplary embodiment, with reference to the following figures:
- Fig. 1: is a cross-sectional view of an implant according to a first embodiment of the present invention;
- Fig. 2: is a cross-sectional view of an implant according to a second embodiment of the present invention;
- Fig. 3: is a cross-sectional view of an implant according to a third embodiment of the present invention;
- Fig. 4: is a cross-sectional view of an implant according to a fourth embodiment of the present invention; and
- Fig. 5: is a cross-sectional view of an implant according to a fifth embodiment of the present invention.

### Detailed description of the preferred embodiments

With reference to the following drawings possible embodiments will be described. The figures and the description show preferred embodiments and shall not be used to limit the invention which is defined by the claims.

Potential applications of the membrane or implant according to the present invention include, but are not limited to, reinforcement and repair of intervertebral disc annular lesions, encapsulation of bone defects, coverage of manibular or maxillar cavities following tooth extraction, ligament and tendon repair, hernia repair, bone fracture fixation, repair of ocular socket injuries or defects, repair of calvaria defects or injuries.

The implant comprises a top surface T being in contact with the organ and a bottom surface B being arranged on the opposite of the top surface T. The bottom surface B is not in contact with the surface of the organ, but it may be in contact with neighbouring tissue, organs etc.

Generally spoken, the top surface T is provided with active properties. The term active properties is to be understood to include at least one of the following:
- adhesive properties and/or properties promoting adhesion
- properties promoting ongrowth and/or ingrowth of proteins, cells and/or biological tissues
- properties promoting electrical conductivity
- anti-bacterial properties

The active properties may be applied to cover the whole top surface T of the implant or they may cover only certain sections of the top surface T. The active properties may be applied according to the embodiments as described herein.

The bottom surface B is provided with non-active properties. Thereby the bottom surface B does not promote adhesion, ingrowth and/or ongrowth. Such a structure having an active surface and a non-active surface is particularly advantageous as the attachment to the organ is enhanced by means of the active top surface T and as the attachment to neighbouring organs or tissue is prevented by means of the non-active bottom surface B.

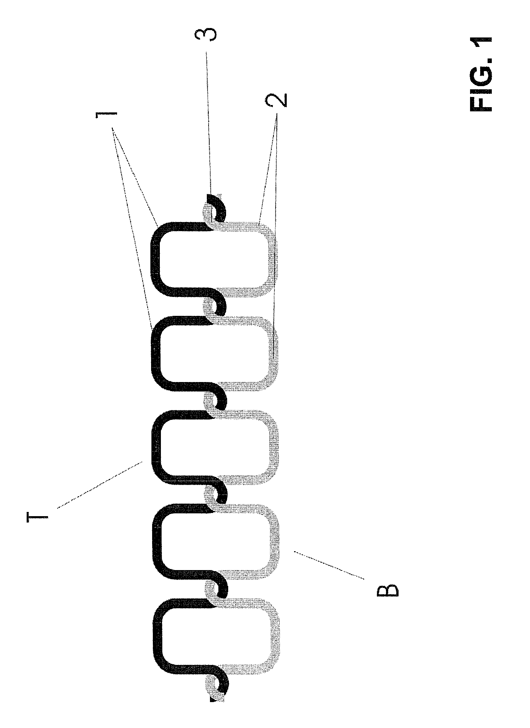

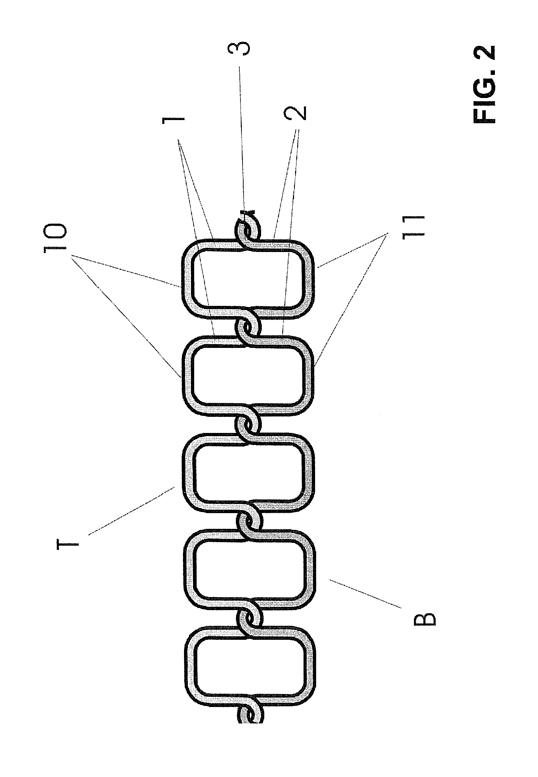

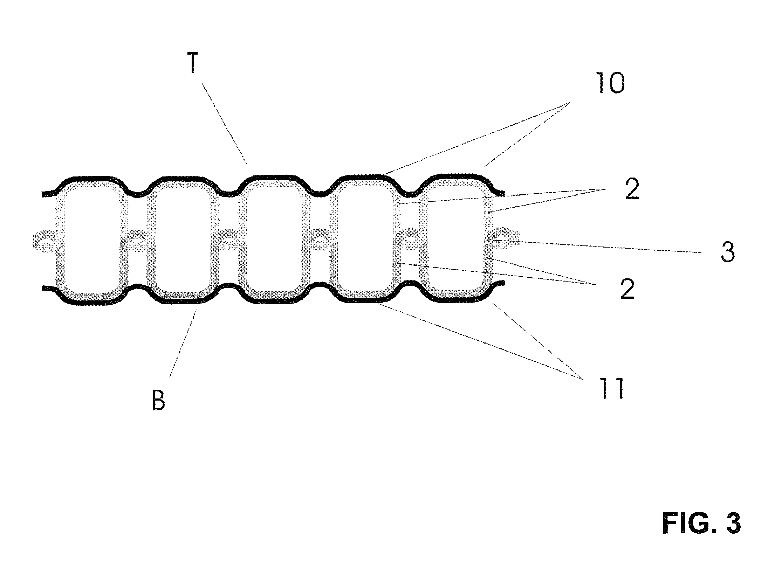

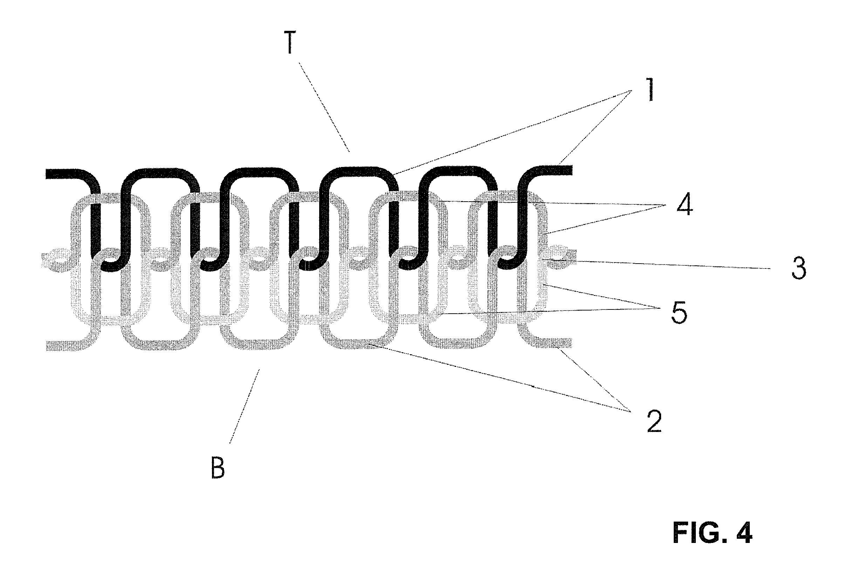

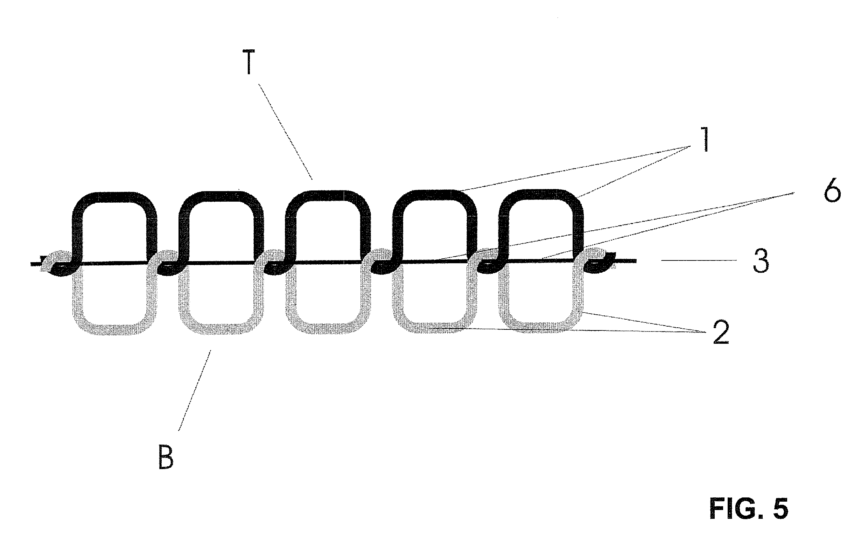

To summarize the properties of the implant or membrane as described herein, one can also say that the adhesive or active properties of one surface of the membrane, herein referred to as the top surface T of the membrane, is accomplished through one of the following production methods:
- An embroidery pattern is so designed that the top-most layer T of the membrane is composed predominantly or exclusively of yam materials which promote adhesion, ongrowth and ingrowth of proteins, cells and/or biological tissues. Such materials include, but are not limited to, titanium filaments and polyester. Such an embodiment is shown in figure 1.
- The embroidery pattern is so designed that the top-most layer of the membrane is composed predominantly or exclusively of yam which has been pre-coated with materials which promote adhesion, ongrowth and ingrowth of proteins, cells and/or biological tissues. Such materials include, but are not limited to, titanium and hydroxyapatite.
   Deposition of such materials is accomplished through coating methods, including but not limited to plasma spraying, solvent coating and electrostatic deposition. The coating method is so designed to produce a uniform layer of adhesion-promoting material while maintaining the intrinsic compliance of the base yarn. Such an embodiment is shown in figure 2.
- An embroidered membrane is composed of one or more conventional yarn materials.
   Following embroidery, the entire top surface of the membrane is coated with materials that promote adhesion, ongrowth and ingrowth of proteins, cells and/or biological tissues.
   Such materials include, but are not limited to, titanium and hydroxyapatite. Deposition of such materials is accomplished through coating methods, including but not limited to plasma spraying, solvent coating and electrostatic deposition. The coating method is so designed to produce a uniform layer of adhesion-promoting material while maintaining the intrinsic compliance of the base membrane. Coating parameters, such as deposited layer thickness, may be specified to either seal or retain the intrinsic pore structure of the embroidered membrane surface. Such an embodiment is shown in figure 3.
- An embroidered membrane is composed of one or more conventional yam materials. The conventional yarns form the structural core of the embroidered membrane. Additional active embroidered layers are interposed in and on this structural core, such that a discrete top surface of the membrane is formed which has properties that promote adhesion, ongrowth and ingrowth of proteins, cells and/or biological tissues. This additional active embroidered layer may be formed from yam materials which include, but are not limited to, titanium filaments and polyester. This additional embroidered layer may also be formed from yam which has been pre-coated with materials which promote adhesion, ongrowth and ingrowth of proteins, cells and/or biological tissues. Such materials include, but are not limited to, titanium and hydroxyapatite. Deposition of such materials is accomplished through coating methods, including but not limited to plasma spraying, solvent coating and electrostatic deposition. The coating method is so designed to produce a uniform layer of adhesion-promoting material while maintaining the intrinsic compliance of the base membrane. Coating parameters, such as deposited layer thickness, may be specified to either seal or retain the intrinsic pore structure of the embroidered membrane surface. Such an embodiment is shown in figure 4.
- An embroidery pattern is so designed that the top-most layer T of the membrane is composed predominantly or exclusively of yam materials which promote adhesion, ongrowth and ingrowth of proteins, cells and/or biological tissues. Such materials include, but are not limited to, titanium filaments and polyester. These active yarn materials are embroidered to a central structural core provided by a woven carrier sheet.
   Such an embodiment is shown in figure 5.

Non-adhesive or non-active properties of one surface of the membrane, opposite to the adhesive surface and herein referred to as the bottom surface B of the membrane, is accomplished through one of the following production methods:
- The embroidery pattern is so designed that the bottom surface B of the membrane is composed predominantly or exclusively of yam materials that inhibit or prevent adhesion, ongrowth and ingrowth of proteins, cells and/or biological tissues. Such materials include, but are not limited to, polyetheretherketone (PEEK) and/or polytetrafluoroethylene (PTFE).
- The embroidery pattern is designed such that the bottom surface B of the membrane is composed predominantly or exclusively of yam which has been pre-coated with materials that inhibit or prevent adhesion, ongrowth and ingrowth of proteins, cells and/or biological tissues. Such materials include, but are not limited to, polyetheretherketone (PEEK) and/or polytetrafluoroethylene (PTFE). Deposition of such materials is accomplished through coating methods, including but not limited to plasma spraying, solvent coating and electrostatic deposition. The coating method is so designed to produce a uniform layer of adhesion-promoting material while maintaining the intrinsic compliance of the base yarn.
- The embroidered membrane is composed of one or more conventional yarn materials. Following embroidery, the entire bottom surface B of the membrane is coated with materials that inhibit adhesion, ongrowth and ingrowth of proteins, cells and/or biological tissues. Such materials include, but are not limited to, polyetheretherketone (PEEK) and/or polytetrafluoroethylene (PTFE). Deposition of such materials is accomplished through coating methods, including but not limited to plasma spraying, solvent coating and electrostatic deposition. The coating method is so designed to produce a uniform layer of adhesion-promoting material while maintaining the intrinsic compliance of the base membrane. Coating parameters, such as deposited layer thickness, may be specified to either seal or retain the intrinsic pore structure of the embroidered membrane surface.
- The embroidered membrane consists of a structural core, composed of one or more conventional yarns. Additional non-active embroidered layers are interposed in and on this structural core, such that a discrete bottom surface B of the membrane is formed which has properties that inhibit adhesion, ongrowth and ingrowth of proteins, cells and/or biological tissues. This interposed non-active layer may be composed predominantly or exclusively of yarn materials that inhibit or prevent adhesion, ongrowth and ingrowth of proteins, cells and/or biological tissues. Such materials include, but are not limited to, polyetheretherketone (PEEK) and/or polytetrafluoroethylene (PTFE). The interposed layer may be composed predominantly or exclusively of yam which has been pre-coated with materials that inhibit or prevent adhesion, ongrowth and ingrowth of proteins, cells and/or biological tissues. Such materials include, but are not limited to, polyetheretherketone (PEEK) and/or polytetrafluoroethylene (PTFE). Deposition of such materials is accomplished through coating methods, including but not limited to plasma spraying, solvent coating and electrostatic deposition. The coating method is so designed to produce a uniform layer of adhesion-promoting material while maintaining the intrinsic compliance of the base yarn.
- The embroidery pattern is so designed that the bottom surface B of the membrane is composed predominantly or exclusively of yam materials that inhibit or prevent adhesion, ongrowth and ingrowth of proteins, cells and/or biological tissues. Such materials include, but are not limited to, polyetheretherketone (PEEK) and/or polytetrafluoroethylene (PTFE). These inhibitory yam materials are embroidered to a central structural core provided by a woven carrier sheet.

Figure 1 shows an implant according to a first embodiment of the present invention in cross-sectional view. The textile implant here comprises an active yam 1 and a non-active yam 2 or carrier yam 2. In this embodiment the active yam 1 provides the top surface T, whereas the carrier yam 2 provides the bottom surface B. The implant according to the present invention comprises therefore an active region provided by the active yarn 1 and a carrier region provided by the carrier yam 2. The active yam 1 is interconnected or embroidered with the carrier yarn 2 in a joining region 3. The joining region 3 in this embodiment extends parallel to the bottom or top surface through the implant. The joining region 3 is arranged such that it is completely inside the implant. This means that the carrier yam 2 extends from the bottom surface B to the joining region 3 and that the active yarn 1 extends from the top surface T to the joining region 3. With other words one can also say that both, the active yarn 1 and the carrier yam 2 extend from their respective surface T, B into the implant but not through the implant.

The active yam 1 and the carrier yam 2 may be provided in the form of a plurality of filling threads which are interconnected with each other, wherein the active properties are applied to the filling threads which are arranged towards the top surface T.

Furthermore, the embroidery pattern may be specified to allocate distinct yarn materials to specific layers throughout the membrane. The membrane comprises a three-dimensional, embroidered structure with the intrinsic characteristic of multiple yam layers having different properties throughout the thickness of the membrane.

In an alternative embodiment the joining region 3 comprises further active substances, such as a medicament, to be delivered slowly via the top surface T to the organ or tissue. Said active substances may be arranged within the three-dimensional embroidered structure.

The top surface T in this embodiment is provided by means of active yam 1 which promotes adhesion, ongrowth and/or ingrowth of proteins, cells and/or biological tissues.

The bottom surface B in this embodiment is provided by means of non-active yarns, i.e. carrier yarn 2. This inhibits adhesion, ongrowth and/or ingrowth between the bottom surface B and the neighbouring tissue or organ.

In an alternative embodiment the same results can be obtained, if a limited number of active yarns compared to whole amount of yarns extend through the implant to the bottom surface B and/or if a limited number of carrier yarns compared to whole amount of yarns extend through the implant to the top surface T. Under a limited number a percentage of less than 30% preferably less than 20% is to be understood.

In an alternative embodiment shown in figure 2 it is also possible to use the first embodiment whereby the top surface T is also provided by means of an active yam 1. The surface of said individual yam 1 is covered by a material 10 promoting adhesion, ongrowth and/or ingrowth of proteins, cells and/or biological tissues. Examples of said materials are named above. The carrier yarn 2, which provides the bottom surface B, is provided with non-active properties. In particular said yam 2 is covered with a non-adhesive coating 11 that inhibits adhesion, ongrowth and/or ingrowth.

Figure 3 shows a third embodiment of an implant or membrane according to the present invention. Same features are designated with same reference numerals. The embroidered or interwoven structure in that embodiment has the same physical structure as the one in the first embodiment. As the top surface T in that embodiment is coated with a layer 10 having active properties, the embroidered structure here is provided with a carrier yarns 2 having non-active properties.

The top surface T is thereby coated with an active material promoting adhesion, ongrowth and/or ingrowth of proteins, cells and/or biological tissues.

Despite the fact that the carrier yarns 2 may have non-adhesive properties, the bottom surface B may optionally also be coated with a non-active coating 11 in order to inhibit adhesion, ongrowth and/or ingrowth between the bottom surface B and the neighbouring tissue or organ. Materials have been mentioned above.

The coating extends over the whole surface of the top surface T or it extends over regions which are parts of the top surface T. This means that the top surface T comprises active and non-active sections. Such a structure prevents unwanted ongrowth or ingrowth in the non-active sections.

The coating is preferably arranged such that it is porous. Thereby the porosity may adjusted to the application of the implant.

In an alternative embodiment it is also possible to use the embodiment of figure 1 and cover the structure with the coatings of figure 3. In a general form one can say that the active yam 1 is covered at least partly or fully by the coating layer 10 having also active properties and/or that the carrier yam 2 is covered by the non-active coating 11. This provides an advantageous structure in particular in terms of adhesion.

Figure 4 shows a fourth embodiment of an implant or membrane according to the present invention. Same features are designated with same reference numerals. In that embodiment, the mechanical competence of the membrane is provided by a central embroidered core 4, 5 and the adhesive and non-adhesive properties of the membrane are provided by additional active layers 1 and non-active layers 2 which are interposed and overlaid on the central core 4, 5.

A structural core is provided by the interconnection of core yarns 4 and 5. The core yarn 4 is interconnected or embroidered with the core yam 5 in a joining region 3.

The top surface T is provided by means of an active yam 1. The surface of said individual yam 1 is preferentially composed of a material promoting adhesion, ongrowth and/or ingrowth of proteins, cells and/or biological tissues or is covered by a material promoting adhesion, ongrowth and/or ingrowth of proteins, cells and/or biological tissues. Examples of said materials are named above.

The carrier yam 2 which provides the bottom surface B is provided with non-active properties. In particular said yam 2 is composed of a material that inhibits adhesion, ongrowth and/or ingrowth or is covered with an non-adhesive coating that inhibits adhesion, ongrowth and/or ingrowth.

The active yam 1 and non-active carrier yard 2 are interconnected or embroidered with the core yarns 4 and 5 in a joining region 3.

In this embodiment all yarns have the same mechanical properties, e.g. the same elastic modulus. In an alternative embodiment, core yarns may have different properties than the active and carrier yarns.

Figure 5 shows a fifth embodiment of an implant or membrane according to the present invention. Same features are designated with same reference numerals. In that embodiment, the mechanical competence of the membrane is provided by a central carrier sheet 6 and the adhesive and non-adhesive properties of the membrane are provided by additional active 1 and non-active layers 2 which are interposed and overlaid on the central sheet. Thereby the carrier sheet 6 provides the joining region 3.

A structural core is provided by a woven carrier sheet 6. This core may be composed of a bioresorbable material, such as, but not limited to, polyglycolic acid (PGA) or polylactic acid (PLA). This core may also possess either permeable or impermeable characteristics.

The top surface T is provided by means of an active yarn 1. The surface of said individual yarn 1 is preferentially composed of a material promoting adhesion, ongrowth and/or ingrowth of proteins, cells and/or biological tissues or is covered by a material promoting adhesion, ongrowth and/or ingrowth of proteins, cells and/or biological tissues. Examples of said materials are named above.

The carrier yarn 2 which provides the bottom surface B is provided with non-active properties. In particular said yarn 2 is composed of a material that inhibits adhesion, ongrowth and/or ingrowth or is covered with an non-adhesive coating that inhibits adhesion, ongrowth and/or ingrowth.

The active yam 1 and non-active carrier yard 2 are interconnected or embroidered with the core carrier sheet in a joining region 3.

The invention comprises a method for producing an embroidered membrane for internal implantation in the body, whereby the production method creates an adhesive surface on one side (i.e. top side T) of the membrane and a non-adhesive surface on the opposite side (i.e. bottom side B). The terms "adhesive" and "non-adhesive" are used in the context of biological adhesion of proteins, cells and/or tissues. Adhesion and non-adhesion of the two membrane surfaces are intrinsic and lasting properties of the membrane and a direct consequence of the specified production methods. Adhesion and non-adhesion properties of the thus-created membrane can be verified using standard in vitro cell or tissue culture assays and conventional histomorphometric or scanning electron microscopic techniques. Adhesion and non-adhesion properties of the thus-created membrane can also be verified using known, conventional in vivo testing methods with subsequent histomorphometric or scanning electron microscopic analysis.

In a further embodiment the implant comprises a structural core having anisotropic mechanical properties. The active and non-active layers as described herein are in connection with said core. Preferably said core is fully encompassed by said layers. Alternatively said core is partly encompassed, e.g. on one or two sides only by said layers. Thereby the core provides the structural mechanical properties such as elasticity, stiffness (in one or more directions), whereas said layers are do not necessarily contribute to the mechanical performance of the implant. Furthermore it is also possible that said core is formed in such a way as to provide the primary control of the implant porosity and/or permeability based on the dimensions of the created pore space in the core, while the attached active / non-active layers determine the surface chemistry of the implant. In the embodiments as just mentioned the structure of the central core region provides control of predominately chemical and biological performance factors.

The implant as described herein may be produced by means shuttle embroidery or shuttle weaving.

List of reference numerals
- 1: active yam
- 2: non-active / carrier yarn
- 3: joining region
- 4: first core yarn
- 5: second core yarn
- 6: carrier sheet
- 10: active coating
- 11: non-active coating

## Claims

1. Internal body implant comprising a three-dimensional textile structure having a top surface (T) and a bottom surface (B), wherein the textile structure comprises multiple yarns being interconnected with each other, **characterized in that** the top surface (T) is provided with active properties, such as adhesive properties and/or properties promoting adhesion and/or properties promoting ongrowth and/or ingrowth of proteins, cells and/or biological tissues and **in that** said active properties are absent at the bottom surface (B).

2. Internal body implant according to claim 1, **characterized in that** the bottom surface (B) is provided with non-active properties.

3. Internal body implant according to one of the preceding claims, **characterized in that** the active properties are applied to at least one section of the top surface (T) wherein said section is defined as having a smaller area than the top surface (T).

4. Internal body implant according to one of the preceding claims, **characterized in that** the active properties are applied to the entire top surface (T).

5. Internal body implant according to one of the preceding claims, **characterized in that** the implant comprises active yarns (1) having said active properties and non-active carrier yarns (2) wherein the active yarn (1) faces the top surface (T) and wherein the carrier yam (2) faces the bottom surface (B).

6. Internal body implant according to one of the preceding claims, **characterized in that** the active yam (1) and the carrier yarn (2) are provided in the form of a plurality of filling threads which are interconnected with each other, wherein the active properties are applied to the filling threads which are arranged towards to top surface (T).

7. Internal body implant according to one of the preceding claims, **characterized in that** the active yarn (1) is composed predominately or exclusively of yam materials that promote adhesion, ongrowth and/or ingrowth of cells and/or biological tissue.

8. Internal body implant according to claim 7, **characterized in that** the active yarn (1) is a titanium filament or a polyester filament.

9. Internal body implant according to one of the preceding claims, **characterized in that** the surface of the active yam (1) is coated with a coating material that promotes adhesion, ongrowth and/or ingrowth of cells and/or biological tissue.

10. Internal body implant according to one of the preceding claims, **characterized in that** the top surface (T) is coated predominately or exclusively with a coating material that promotes adhesion, ongrowth and/or ingrowth of cells and/or biological tissue.

11. Internal body implant according to claim 9 or 10, **characterized in that** the coating material is titanium or hydroxyapatite.

12. Internal body implant according to one of the preceding claims, **characterized in that** the non-active yarn (2) is composed predominately or exclusively of yarn materials that inhibits adhesion, ongrowth and/or ingrowth of cells and/or biological tissue.

13. Internal body implant according to one of the preceding claims, **characterized in that** the surface of the non-active yarn (2) is coated with a coating material that inhibits adhesion, ongrowth and/or ingrowth of cells and/or biological tissue.

14. Internal body implant according to one of the preceding claims, **characterized in that** the bottom surface (B) is coated predominately or exclusively with a coating material that inhibits adhesion, ongrowth and/or ingrowth of cells and/or biological tissue.

15. Internal body implant according to one of the claims 12 to 14, **characterized in that** the material that inhibits adhesion, ongrowth and/or ingrowth is polyetheretherketone or polytetrafluoroethylene.

16. Internal body implant according to one of the preceding claims, **characterized in that** the implant comprises a core section (3, 4, 5, 6) being encompassed by the active yam (1) and/or by the non-active yarn (2), wherein the core section (3, 4, 5, 6) comprises several yarns (4, 5) and/or a carrier sheet (6).
